# EUROPÄISCHE PATENTSCHRIFT

(11) **EP 2 544 000 B1**
(45) Veröffentlichungstag und Bekanntmachung des Hinweises auf die Patenterteilung: **10.08.2016**
(21) Anmeldenummer: 12160562.0
(22) Anmeldetag: 21.03.2012
(51) Int. Cl.: A61B 5/00, G01N 33/00, A61B 5/08, G01N 21/3504, G01N 21/03, G01N 21/05, G01N 33/497

(54) **Atemalkoholgehaltmessgerät**
Breath alcohol measurement device
Éthylomètre

(30) Priorität: 02.07.2011 DE 102011106410
(43) Veröffentlichungstag der Anmeldung: 09.01.2013
(73) Patentinhaber: Dräger Safety AG & Co. KGaA, 23560 Lübeck (DE)
(72) Erfinder: Stock, Burkhard, 23564 Lübeck (DE)

(56) Entgegenhaltungen:
- WO-A1-2005/051700
- WO-A1-2007/083350
- WO-A1-2011/042679
- DE-C1- 19 913 783
- US-A- 4 017 792
- US-A1- 2008 200 824

## Beschreibung

Die Erfindung betrifft ein Atemalkoholgehaltmessgerät mit einem Grundkörper enthaltend einen Durchflusskanal für den Atemluftstrom, mit einem den Atemluftstrom einer Person aufnehmenden austauschbaren Mundstück, das an einem stromaufwärts gelegenen Ende des Durchflusskanals mit dem Grundkörper verbunden Ist, mit einer dem Grundkörper zugeordneten Alkoholsensoreinhelt zur Messung des Alkoholgehaltes des Atemluftstromes und einer Temperatursensoreinhelt zur Messung einer Temperatur des Atemluftstromes, mit einer Heizelnhelt zum Aufhelzen des Durchflusskanals, mit einer Steuereinhelt zur Verarbeitung von durch die Temperatursensoreinhelt bereitgestellten Messsignalen und zur Ansteuerung der Helzeinhelt.

Aus der DE 198 11 177 C2 und DE 199 13 783 C1 ist jeweils ein Atemalkoholgehaltmessgerät bekannt, das über einen Grundkörper mit einem Durchflusskanal verfügt, durch den ein Atemluftstrom durchgeleitet und mittels einer Sensoreinheit auf Alkoholgehalt gemessen wird. Dieser Atemluftstrom wird üblicherweise als eine Atemluftprobe von einer Person über ein an dem Grundkörper befesligbares und austauschbares Mundslück abgegeben. Um ein korrektes Massergebnis zu erzielen, Insbesondere zur Vermeldung von Kondensation des Atemluftstroms bei niedrigen Temperaturen, weist das Gerät eine Heizeinhelt auf, mittels derer der Grundkörper In einem zu dem Durchflusskanal nahen Bereich aufgewärmt wird. Hierzu Ist eine Drahtwendel in dem Grundkörper eingebettet, deren Anschlüsse nach außen geführt sind, um mit einem elektrischen Strom gespeist zu worden. Ferner weist das Atemalkoholgehaltmessgerät eine Sensoreinhelt zur Messung der Temperatur des Atemluftstroms bzw. des Mundstückes auf, so dass Im Zusammenwirken mit einer Steuereinheit Alkoholmessproben erfasst und ausgewertet werden können, wenn ein vorgegebener Mindesttemperaturwert in dem Durchflusskanal vorliegt. Nachteilig an dem bekannten Atemalkoholgehaltmesagerät ist, dass die Aufhelzung des Durchflusskanals Infolge des punktuellen Wärmeeintrags mittels der Drahtwendel In den Grundkörper relativ träge ist.

Aufgabe der vorliegenden Erfindung Ist es daher, ein Atemalkoholgehaltmassgerät derart weiterzubilden, dass die Messung des Alkoholgehaltes in einem Atemluftstrom einer Person relativ schnell unter Aufwendung einer relativ geringen Heizleistung gewährleistet ist.

Zur Lösung dieser Aufgabe Ist die Erfindung In Verbindung mit dem Oberbegriff des Patentanspruchs 1 dadurch gekennzeichnet, dass der Grundkörper aus einem elektrisch leitenden Polymermaterial besteht und dass eine Anzahl von Kontaktelektroden an dem Grundkörper anliegen und/oder in diesen eingebettet sind wobei die Kontaktelektroden jeweils über elektrische Leitungen mit elektrischem Strom versorgbar sind.

Der besondere Vorteil der Erifndung besteht darin, dass durch die Ausbildung des Grundkörpers aus einem elektrisch leitenden Polymermaterial eine relativ schnelle und homogene Erwärmung des Durchflusskanals bzw. des Mundstücks gewährleisiet. Der Kondensation des Atemluftstroms wird effektiv entgegengewirkt, so dass auch bei niedrigen Temperaturen das Atemalkoholgehaltmessgerät relativ schnell aktivierbar Ist. Insbesondere bei sogenannten "Interlock"-Systemen in Kraftfahrzeugen, bei denen der Start des Kraftfahrzeugs den erfolgreichen Abschluss eines Atemalkcholtestes voraussetzt, wirkt sich die kürzere Wartezeit bis zur Betriebsfähigkeit des Messgerätes günstig aus.

Nach einer bevorzugten Ausführungsform der Erfindung sind mit einer elektrischen Spannungsquelle verbundene Kontaktelektroden verteilt an dem Grundkörper angeordnet und/oder In dieselben eingebettet. Vorteilhaft kann hierdurch eine gleichmäßige und homogene Wärmeeinbringung in den Grundkörper bzw. in die Sensoreinheit erfolgen.

Nach einer Weiterbildung der Erfindung werden die Kontaktelektroden In Abhängigkeit von in der Sensoreinheit bereitgestellten Temperaturmesssignalen derart mit einer elektrischen Stromstärke beaufschlagt, dass der von der Sensoreinheit gemessene Temperaturwert des Grundkörpers und/oder der Sensoreinheit gleich oder größer ist als ein vorgegebener Mindesttemperaturwert. Hierdurch wird sichergestellt, dass ein Atemluftalkoholtest nur durchgeführt wird, wenn die Voraussetzungen für ein korrektes Messergebnis vorliegen. Dadurch, dass mehrere Kontaktelektroden paarweise angesteuert werden können, kann ortsabhängig ein Ternperaturausgleich erfolgen. Auf unterschiedliche Randbedingungen bzw. Temperaturprofile kann somit im Sinne einer Homogenisierung der Atemlufttemperatur eingewirkt werden.

Nach einer Weiterbildung der Erfindung kann das Polymermaterial des Grundkörper. mit einem elektrisch leitenden Füllstoff versehen oder elektrisch selbstleitend ausgebildet sein, um die erforderliche elektrische Leitfähigkeit zu erhalten.

Durch die Verwendung des elektrisch leitenden Füllstoffs oder des elektrisch leitenden Polymermaterials ergibt sich neben der elektrischen Leitfähigkeit zugleich eine gute thermische Leitfähigkeit, da elektrisch leitfähige Materialien prinzipiell auch eine gute Wärmeleitfähigkeit aufweisen.

Vorteilhaft kann hierdurch unter Einsatz einer relativ geringen elektrischen Leistung eine schnelle Aufheizung des zu messenden Atemluftstroms erreicht werden.

Weitere Vorteile der Erfindung ergeben sich aus den weiteren Unteransprüchen.

Ausführungsbeispiele der Erfindung werden anhand der Zeichnungen näher erläutert.

Es zeigen:

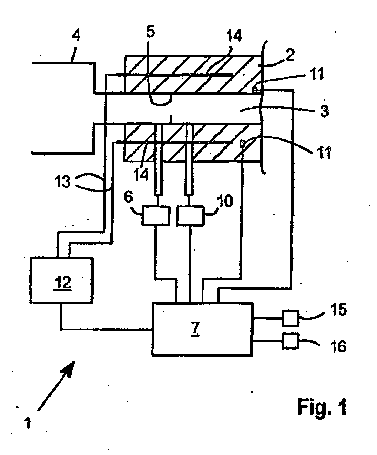

- Fig. 1: eine schematische Darstellung eines Atemalkoholgehaltmessgerät nach einer ersten Ausführungsform und
- Fig. 2: eine schematische Darstellung des Atemalkoholgehaltmessgeräts nach einer zweiten Ausführungsform.

Ein Atemalkoholgehaltmessgerät 1 kann als mobiles Messgerät für Alkoholkontrollen im Straßenverkehr oder als stationäres Gerät ("interlock"-Gerät) fest installiert in einem Kraftfahrzeug ausgebildet sein.

Das Messgerät 1 weist einen Grundkörper 2 auf, der einen Durchflusskanal 3 zur Durchleitung eines von einer Person abgegebenen Atemluftstroms aufweist. An einem stromaufwärts gelegenen Ende des Durchflusskanals 3 ist der Grundkörper 2 mit einem austauschbaren Mundstück 4 verbunden, durch das die Person seine Atemluftstrom einbläst. In dem Durchflusskanal 3 ist eine Strömungsblende 5 mit einem kreisförmigen Querschnitt und einem Durchmesser von 3 mm bis 4 mm angeordnet, mittels derer ein Druckabfall des Atemluftstroms bewirkt wird. Der Druckabfall wird mittels eines Drucksensors 6 gemessen, der die Druckmessdaten einer Steuereinheit 7 zur Verfügung stellt.

Eine Temperatursensoreinheit umfasst mindestens einen Temperaturmesswiderstand 11, der in dem Grundkörper 2 eingefasst ist zur Bestimmung der Temperatur des Atemluftstromes und/oder der Temperatur des Grundkörpers 2 und der ein entsprechendes Temperatursignal an die Steuereinheit 7 liefert. Liegt der von der Temperatursensoreinheit für die Steuereinheit 7 bereitgestellte Temperaturmesswert unterhalb eines Mindesttemperaturwertes, wird mittels der Steuereinheit 7 eine Heizeinheit 12 angesteuert zum Aufheizen des Durchflusskanals 3 bzw. des Grundkörpers 2. Die Heizeinheit 12 weist eine mit einer Stromversorgungseinheit verbundene gesteuerte Spannungs- oder Stromquelle auf und ist über elektrische Leitungen 13 mit an dem Grundkörper 2 anliegenden und/oder in demselben eingebetteten Kontaktelektroden 14 verbunden. Vorzugsweise sind die Kontaktelektroden 14 so verteilt innerhalb des Grundkörpers 2 angeordnet, dass durch Ansteuerung der Kontaktelektroden 14 eine homogene Erwärmung des Grundkörpers 2 bzw. des Durchflusskanals 3 gewährleistet ist. Zu diesem Zweck können die Kontaktelektroden 14 stirnseitig des Grundkörpers 2 in denselben über die gesamte Länge eingreifen. Alternativ können die stabförmigen Kontaktelektroden 14 auch quer zum Durchflusskanal 3 von einer Mantelfläche des Grundkörpers 2 her in denselben eingebettet sein.

Damit der Grundkörper 2 homogen erwärmt wird, besteht der Grundkörper 2 aus einem elektrisch leitenden Polymermaterial, das beispielsweise mit einem elektrisch leitenden Füllstoff, insbesondere Ruß oder Kaminruß, Kohlenstofffasern und/oder Graphitpulver und/ oder Metallpulver gefüllt sein kann.
Der Füllstoff kann beispielsweise zu einem wärmehärtenden oder thermoplastischen Harz gegeben sein.

Das Polymermaterial des Grundkörpers 2 ist vorzugsweise derart ausgebildet, dass sich zwischen zwei Kontaktelektroden 14 ein ohmscher Widerstand in einem Bereich zwischen 10 Ω und 50 Ω, vorzugsweise zwischen 10 Ω und 20 Ω einstellt.
Der elektrische Volumenwiderstand kann im Bereich zwischen 900 Ωcm bis 1200 Ωcm, vorzugsweise 1100 Ωcm, die thermische Leitfähigkeit in einem Bereich zwischen 10 W/mK und 50 W/mK, vorzugsweise 20 W/mK liegen.

Damit ein korrektes Alkoholgehaltmessergebnis erzielt werden kann, muss vor dem Atemtest der Durchflusskanal 3 auf eine Temperatur gebracht werden, die über der Kondensationstemperatur des Atemluftstromes liegt, also über 35° C.

Die Steuereinheit 7 ist mit einer Anzeige 15 zur Darstellung messbezogener Daten für die die Messung durchführende Person sowie mit Bedienelementen 16 zum Ein- und Ausschalten des Messgerätes 1 gekoppelt.

Soll eine Messung des Atemalkoholgehaltes erfolgen, wird mittels des Bedienelementes die Steuereinheit 7 eingeschaltet. Nachfolgend wird mittels der Temperatursensoreinheit die aktuelle Temperatur des Grundkörpers 2 ermittelt. Liegt diese oberhalb des vorgegebenen Mindesttemperaturwertes, erhält die Heizeinheit 12 kein die Kontaktelektroden 14 einschaltendes Steuersignal. Ist die aktuelle Temperatur unterhalb des Mindesttemperaturwertes, erhält die Heizeinheit 1 von der Steuereinheit 7 ein die Kontaktelektroden 14 einschaltendes Steuersignal, damit der Grundkörper 2 so lange aufgeheizt wird, bis die erforderliche Mindesttemperatur erreicht ist. Nachfolgend wird die betreffende Person aufgefordert, einen Atemtest abzugeben. Hierbei strömt die Atemluft durch das Mundstück 4 in den Durchflusskanal 3. Den entstehenden Staudruck erfasst der Drucksensor 6, dessen Ausgangssignal von der Steuereinheit 7 bei der Atemluftabgabe kontinuierlich in das von der Person abgegebene Volumen umgerechnet wird. Wenn das Ausgangssignal einen Schwellwert überschritten hat, wird über die Steuereinheit 7 eine nicht dargestellte Pumpe gestartet und eine Probe aus dem Atemluftstrom zu dem Alkoholsensor der Alkoholgehaltsensoreinheit 10 gezogen. Aus dem von dem Alkoholsensor 10 bereitgestellten Messsignal wird in der Steuereinheit 7 nach einem bekannten Verfahren der Alkoholgehalt bestimmt. Der gemessene Atemalkoholgehalt wird dann in der Anzeige 15 dargestellt.

Die Sensoreinheiten 6, 10, 11 sind vorzugsweise unabhängig voneinander und direkt mit der Steuereinheit 7 elektrisch verbunden.

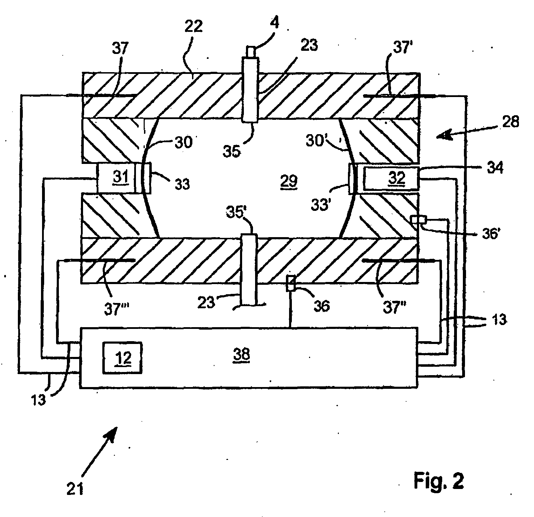

Nach einer weiteren Ausführungsform (Fig. 2) der Erfindung kann ein Atemalkoholgehaltmessgerät 21 vorgesehen sein, das im Unterschied zu dem vorhergehenden Meßgerät 1 nicht einen elektrochemischen Alkoholgehaltsensor 10, sondern einen Infrarotsensor 28 aufweist.

Gleiche Bauteile bzw. Bauteilfunktionen der Messgeräte 1, 21 sind mit den gleichen Bezugsziffern versehen.

Der Infrarotsensor 28 ist in einem als eine Küvette ausgebildeten Grundkörper 22 mit einem Durchflusskanal 23 integriert angeordnet. Das Mundstück 4 Ist an einem stromaufwärts gelegenen Ende des Durchflusskanals 23 lösbar mit dem Grundkörper 22 verbunden.

Der Infrarotsensor 28 umfasst zwei in einem Messraum 29 gegenüberliegend angeordnete Spiegel 30, 30' auf, die in dem Grundkörper 22 eingefasst sind. Innerhalb des einen Spiegels 30 ist eine Infrarotstrahlungsquelle 31 und innerhalb des Spiegels 30' ein Strahlungsdetektor 32 angeordnet. Der Abstand zwischen den Spiegeln 30, 30' sowie Brennweiten derselben sind so gewählt, dass die Infrarotstrahlungsquelle 31 auf den Strahlungsdetektor 32 nach mehrmaligen Strahlungsdurchgängen, beispielsweise drei Durchgängen, abgebildet wird. Beispielsweise kann der Abstand der Spiegel 30, 30', zueinander 5 cm betragen, so dass die optische Weglänge hier bei 15 cm liegt.

Die Infrarotstrahlungsquelle 31. und der Strahlungsdetektor 32 sind jeweils durch strahlungsdurchlässige Fenster 33 bzw. 33' von dem den Atemluftstrom führenden Durchflusskanal 23 bzw. Messraum 29 abgedichtet.

Vorzugsweise ist der Strahlungsdetektor 32 in einem gesonderten Halter 34 angeordnet, der von dem Grundkörper 22 thermisch entkoppelt ist. Temperaturänderungen des Grundkörpers 22 wirken sich somit nicht auf den thermischen Strahlungsdetektor 32 aus.

Über einen Gaseinlass 35 und einen Gasauslass 35' strömt die Atemluftprobe In den Messraum 29 des Infrarotsensors 28.

Die mit einer Steuereinheit 38 verbundene Temperatursensoreinheit umfasst zwei Temperatursensoren 36, 36', wobei der eine Temperatursensor 36 in dem Spiegel 30' und der andere Temperatursensor 36' an dem Grundkörper 22 eingefasst ist.

Die in der Steuereinheit 38 integrierte Heizeinheit 12 ist über die elektrischen Leitungen 13 mit vier Kontaktelektroden 37, 37', 37", 37"' verbunden, die verteilt in dem Grundkörper 22 eingebettet sind.

Durch Ansteuerung der Heizeinheit 12 mittels der Steuereinheit 7 werden die Kontaktelektroden 37, 37', 37", 37'" paarweise gleichzeitig oder sequentiell mit einer vorgegebenen elektrischen Spannung beaufschlagt. Beispielsweise können die Kontaktelektroden 37 und 37" mit einem Pluspol der Spannungsquelle und die Kontaktelektroden 37' und 37"' mit einem Minuspol der Spannungsquelle verbunden sein. Der Heizstrom fließt dann durch den Grundkörper 22 zwischen den Kontaktelektroden 37 und 37', 37 und 37"', 37"' und 37" sowie 37' und 37". Mittels der Temperatursensoren 36, 36' wird die aktuelle Temperatur während des Heizvorganges ermittelt und durch wahlweises Ab- und Einschalten der Kontaktelektroden 37, 37', 37", 37'" die Energieverteilung bzw. der Wärmeeintrag in dem Grundkörper 22 bzw. dem Infrarotsensor 28 so gesteuert, dass beide Temperatursensoren 36, 36' im wesentlichen die gleiche Temperatur messen, die einer Solltemperatur entsprechen soll. Durch das wahlweise Ein-/Abschalten von Paaren von Kontaktelektroden 37, 37', 37", 37'" können Temperaturgradienten innerhalb des Grundkörpers 22 bzw. des Infrarotsensors 28 ausgeglichen werden.

Nach einer nicht dargestellten alternativen Ausführungsform können die Kontaktelektroden 37,37', 37", 37"' auch so positioniert werden, dass zwischen denselben stets der gleiche ohmsche Widerstand in dem Grundkörper 22 bzw. der Infrarotsensor 28 besteht.

Zur Messung des Atemalkoholgehaltes wird das Gerät mittels des Bedienelementes 16 eingeschaltet, so dass zunächst der Grundkörper 22 mittels der Heizeinheit 12 aufgeheizt wird. Wenn der Grundkörper 22 auf ca. 40° C aufgeheizt ist, wird mittels einer nicht dargestellten Pumpe Umgebungsluft in den Messraum 29 gezogen. Das sich dabei einstellende Signal des Strahlungsdetektors 32 wird in der Steuereinheit 7 als Referenzwert abgespeichert. Dann wird die Person aufgefordert eine Atemprobe abzugeben. Der Atemluftstrom gelangt dann über den Anschluss 35 in den Messraum 29. Aus dem dann gemessenen Messwert des durch den Strahlungsdetektor 32 gemessenen Messwertes und den zuvor ermittelten Referenzwert errechnet die Steuereinheit 7 die Alkoholkonzentration. Das Messergebnis wird in der Anzeige 15 dargestellt. Nach Beendigung des Messvorgangs wird der Messraum 29 zur Säuberung wieder mit Umgebungsluft gespült.

### Bezugszeichenliste

- 1: Atemalkoholgehaltmessgerät
- 2: Grundkörper
- 3: Durchflusskanal
- 4: Mundstück
- 5: Strömungsblende
- 6: Drucksensor
- 7: Steuereinheit
- 10: Alkoholgehaltsensorseinheit
- 11: Temperaturmesswiderstand
- 12: Heizeinheit
- 13: elektrische Leitungen
- 14: Kontaktelektroden
- 15: Anzeige
- 16: Bedienelement
- 21: Atemalkoholgehaltmessgerät
- 22: Grundkörper
- 23: Durchflusskanal
- 28: Infrarotsensor
- 29: Messraum
- 30, 30': Spiegel
- 31: Infrarotstrahlungsquelle
- 32: Strahlungsdetektor
- 33, 33': Fenster
- 34: Halter
- 35: Gaseinlass
- 35': Gasauslass
- 36, 36': Temperatursensor
- 37, 37', 37", 37"': Kontaktelektroden
- 38: Steuereinheit

## Patentansprüche

1. Atemalkoholgehaltmessgerät (1, 21) mit einem Grundkörper (2, 22) enthaltend einen Durchflusskanal (3) für einen Atemluftstrom, mit einem den Atemluftstrom einer Person aufnehmenden austauschbaren Mundstück (4), das an einem stromaufwärts gelegenen Ende des Durchflusskanals mit dem Grundkörper verbunden ist, mit einer dem Grundkörper zugeordneten Alkoholsensoreinheit (10, 28) zur Messung des Alkoholgehaltes des Atemluftstromes und einer Temperatursensoreinheit (12, 36, 36') zur Messung einer Temperatur des Atemluftstromes, mit einer Heizeinheit (12) zum Aufheizen des Durchflusskanals, mit einer Steuereinheit (7, 38) zur Verarbeitung von durch die Temperatursensoreinheit bereitgestellten Messsignalen und zur Ansteuerung der Heizeinheit, **dadurch gekennzeichnet, dass** der Grundkörper (2, 22) aus einem elektrisch leitenden Polymermaterial besteht und dass eine Anzahl von Kontaktelektroden (14, 37, 37', 37", 37"') zur Erwärmung des Durchflusskanals (3, 23) an dem Grundkörper (2, 22) anliegen und/oder in demselben eingebettet sind, wobei die Kontaktelektroden (14, 37, 37', 37", 37"') jeweils, über elektrische Leitungen (13) mit elektrischem Strom speisbar sind.

2. Atemalkoholgehaltmessgerät nach Anspruch 1, **dadurch gekennzeichnet, dass** die Kontaktelektroden (14, 37, 37', 31", 37'") verteilt an dem Grundkörper (2, 22) angeordnet sind.

3. Atemalkoholgehaltmessgerät nach Anspruch 1 oder 2, **dadurch gekennzeichnet, dass** die Kontaktelektroden (14, 37, 37', 37'', 37''') in Abhängigkeit von den durch die Temperatursensoreinheit (11, 36, 36') bereitgestellten Temperaturmesssignalen derart wahlweise mit einer solchen elektrischen Stromstärke speisbar sind, dass die von der Temperatursensoreinheit (11, 36, 36') gemessenen Temperaturwerte des Grundkörpers (2, 22) und/oder der Alkoholgehaltsensoreinheit (10, 28) gleich oder größer als ein vorgegebener Mindesttemperaturwert sind.

4. Atemalkoholgehaltmessgerät nach einem der Ansprüche 1 bis 3, **dadurch gekennzeichnet, dass** die Temperatursensoreinheit (11, 36, 36') mindestens zwei Temperaturmesswiderstände (11) umfasst, die jeweils an dem Grundkörper (2, 22) angeordnet sind, und dass die Kontaktelektroden (14, 37, 37', 37'', 37''') mit einer solchen elektrischen Stromstärke speisbar sind, dass die Temperaturmesswiderstände (11) die gleichen Temperaturmesssignale liefern.

5. Atemalkoholgehaltmessgerät nach einem der Ansprüche 1 bis 4, **dadurch gekennzeichnet, dass** die Kontaktelektroden (14, 37, 37', 37'', 37''') paarweise an dem Grundkörper (2, 22) derart eingebettet sind, dass sich zwischen dem Paar von Kontaktelektroden (14, 37, 37', 37", 37''') ein ohmscher Widerstand in dem Bereich von 10 Ω bis 50 Ω, vorzugsweise zwischen 10 Ω und 20 Ω, einstellt.

6. Atemalkoholgehaltmessgerät nach einem der Ansprüche 1 bis 5, **dadurch gekennzeichnet, dass** das Polymermaterial des Grundkörpers (2, 22) mit einem elektrisch leitenden Füllstoff, insbesondere Ruß oder Kaminruß, Kohlenstofffaser und/oder Grafitpulver und/oder Metallpulver gefüllt ist.

7. Atemalkoholgehaltmessgerät nach einem der Ansprüche 1 bis 6, **dadurch gekennzeichnet, dass** das Polymermaterial des Grundkörpers (2, 22) derart ausgebildet ist, dass sich zwischen zwei beabstandet zueinander angeordneten Kontaktelektroden (14, 37, 37', 37'', 37''') ein Volumenwiderstand zwischen 900 Ωcm und 1.200 Ωcm und/oder eine thermische Leitfähigkeit im Bereich zwischen 10 W/mK und 50 W/mK einstellt.

## Claims

1. A breath alcohol content measuring device (1, 21) having a base body (2, 22) containing a through-flow channel (3) for a respiratory air flow, having an exchangeable mouth piece (4) that receives the respiratory air flow of a person and is connected to the base body at an end of the through-flow channel that is situated upstream, having an alcohol sensor unit (10, 28), which is associated with the base body, to measure the alcohol content of the respiratory air flow and a temperature sensor unit (11, 36, 36') to measure a temperature of the respiratory air flow, having a heating unit (12) to heat up the through-flow channel, having a control unit (7, 38) to process measurement signals provided by the temperature sensor unit and to activate the heating unit, **characterised in that** the base body (2, 22) consists of an electrically conductive polymer material, and **in that** a number of contact electrodes (14, 37, 37', 37'', 37''') for heating the through-flow channel (3, 23) rest against the base body (2, 22) and/or are embedded in the same, wherein the contact electrodes (14, 37, 37', 37'', 37''') can be fed with electric current in each case by way of electrical lines (13).

2. A breath alcohol content measuring device according to claim 1, **characterised in that** the contact electrodes (14, 37, 37', 37'', 37''') are arranged on the base body (2, 22) in a distributed manner.

3. A breath alcohol content measuring device according to claim 1 or 2, **characterised in that** the contact electrodes (14, 37, 37', 37'', 37''') can be selectively fed with such an electric current intensity, as a function of the temperature-measurement signals provided by the temperature sensor unit (11, 36, 36'), so that the temperature values of the base body (2, 22) and/or of the alcohol content sensor unit (10, 28) measured by the temperature sensor unit (11, 36, 36') are equal to or greater than a given minimum temperature value.

4. A breath alcohol content measuring device according to one of claims 1 to 3, **characterised in that** the temperature sensor unit (11, 36, 36') comprises at least two temperature-measuring resistors (11), which in each case are arranged at the base body (2, 22), and **in that** the contact electrodes (14, 37, 37', 37'', 37''') can be fed with such an electric current intensity that the temperature-measuring resistors (11) deliver the same temperature-measurement signals.

5. A breath alcohol content measuring device according to one of claims 1 to 4, **characterised in that** the contact electrodes (14, 37, 37', 37'', 37''') are embedded in pairs at the base body (2, 22) in such a way that an ohmic resistance sets in between the pair of contact electrodes (14, 37, 37', 37'', 37''') in the range of 10 Ω and 50 Ω, preferably between 10 Ω and 20 Ω.

6. A breath alcohol content measuring device according to one of claims 1 to 5, **characterised in that** the polymer material of the base body (2, 22) is filled with an electrically conductive filler, in particular soot or chimney soot, carbon fibre and/or graphite powder and/or metal powder.

7. A breath alcohol content measuring device according to one of claims 1 to 6, **characterised in that** the polymer material of the base body (2, 22) is formed in such a way that between two contact electrodes (14, 37, 37', 37'', 37''') that are arranged so as to be spaced apart from each other a volume resistivity of between 900 Ωcm and 1,200 Ωcm and/or a thermal conductivity in the range between 10 W/mK and 50 W/mK set/sets in.

## Revendications

1. Ethylomètre (1, 21) comprenant un corps de base (2, 22) intérieurement muni d'un canal d'écoulement (3) destiné à un flux d'air respiratoire ; un embout (4) remplaçable, qui reçoit le flux d'air respiratoire d'une personne et est relié audit corps de base à une extrémité dudit canal d'écoulement située en amont ; une unité (10, 28) de détection d'alcool, associée audit corps de base et dévolue à la mesure du taux d'alcool du flux d'air respiratoire, et une unité (11, 36, 36') de détection de températures, dévolue à la mesure d'une température dudit flux d'air respiratoire ; une unité de chauffage (12) pour chauffer ledit canal d'écoulement ; et une unité de commande (7, 38) affectée au traitement de signaux de mesure produits par ladite unité de détection de températures, et au pilotage de ladite unité de chauffage, **caractérisé par le fait que** le corps de base (2, 22) est constitué d'un matériau polymère électriquement conducteur ; et **par le fait qu'**un certain nombre d'électrodes de contact (14, 37, 37', 37", 37"'), dédiées au chauffage du canal d'écoulement (3, 23), sont appliquées sur ledit corps de base (2, 22) et/ou sont intégrées dans ce dernier, lesdites électrodes de contact (14, 37, 37', 37", 37"') pouvant être alimentées en courant électrique par l'intermédiaire de conducteurs électriques (13) respectifs.

2. Ethylomètre selon la revendication 1, **caractérisé par le fait que** les électrodes de contact (14, 37, 37', 37", 37"') sont agencées avec répartition sur le corps de base (2, 22).

3. Ethylomètre selon la revendication 1 ou 2, **caractérisé par le fait que** les électrodes de contact (14, 37, 37', 37", 37"') peuvent être sélectivement alimentées, en fonction des signaux de mesure de température produits par l'unité (11, 36, 36') de détection de températures, en un courant électrique d'une intensité telle que les valeurs de température du corps de base (2, 22) et/ou de l'unité (10, 28) de détection de taux d'alcool, mesurées par ladite unité (11, 36, 36') de détection de températures, soient égales ou supérieures à une valeur de température minimale préétablie.

4. Ethylomètre selon l'une des revendications 1 à 3, **caractérisé par le fait que** l'unité (11, 36, 36') de détection de températures inclut au moins deux résistances (11) de mesure de températures, respectivement implantées sur le corps de base (2, 22) ; et **par le fait que** les électrodes de contact (14, 37, 37', 37", 37"') peuvent être alimentées en un courant électrique d'une intensité telle que lesdites résistances (11) de mesure de températures délivrent les mêmes signaux de mesure de température.

5. Ethylomètre selon l'une des revendications 1 à 4, **caractérisé par le fait que** les électrodes de contact (14, 37, 37', 37", 37"') sont intégrées dans le corps de base (2, 22) de manière appariée, de façon telle qu'une résistance ohmique située dans la plage de 10 Ω à 50 Ω, de préférence comprise entre 10 Ω et 20 Ω, s'instaure entre la paire d'électrodes de contact (14, 37, 37', 37", 37"').

6. Ethylomètre selon l'une des revendications 1 à 5, **caractérisé par le fait que** le matériau polymère du corps de base (2, 22) est garni d'une substance de charge électriquement conductrice, en particulier de la suie ou du noir de fumée, de la fibre de carbone et/ou de la poudre de graphite et/ou de la poudre de métal.

7. Ethylomètre selon l'une des revendications 1 à 6, **caractérisé par le fait que** le matériau polymère du corps de base (2, 22) est réalisé de façon telle qu'une résistivité volumique comprise entre 900 Ωcm et 1 200 Ωcm, et/ou une conductivité thermique située dans la plage comprise entre 10 W/mK et 50 W/mK, s'instaure(nt) entre deux électrodes de contact (14, 37, 37', 37", 37"') placées à distance l'une de l'autre.
